# EUROPÄISCHE PATENTSCHRIFT

(11) **EP 2 318 351 B1**
(45) Veröffentlichungstag und Bekanntmachung des Hinweises auf die Patenterteilung: **30.11.2011**
(21) Anmeldenummer: 09777203.2
(22) Anmeldetag: 15.07.2009
(51) Int. Cl.: C07C 51/235

(54) **VERFAHREN ZUR HERSTELLUNG VON ARYLPOLYGLYKOLCARBONSÄUREN MITTELS DIREKTOXIDATION**
METHOD FOR THE PRODUCTION OF ARYL POLYGLYCOL CARBOXYLIC ACIDS BY MEANS OF A DIRECT OXIDATION PROCESS
PROCÉDÉ DE PRODUCTION D'ACIDES ARYL-POLYGLYCOL-CARBOXYLIQUES PAR OXYDATION DIRECTE

(30) Priorität: 08.08.2008 DE 102008037065
(43) Veröffentlichungstag der Anmeldung: 11.05.2011
(73) Patentinhaber: Clariant Finance (BVI) Limited, Road Town, Tortola (VG)
(72) Erfinder: FRANKE, Oliver, 81671 München (DE); STANKOWIAK, Achim, 84503 Altötting (DE); KUPFER, Rainer, 65795 Hattersheim (DE); PRÜSSE, Ulf, 38118 Braunschweig (DE); DECKER, Nadine, 65835 Liederbach a. Ts. (DE); VORLOP, Klaus-Dieter, 38102 Braunschweig (DE)
(74) Vertreter: Mikulecky, Klaus
(86) Internationale Anmeldenummer: PCT/EP2009/005134
(87) Internationale Veröffentlichungsnummer: WO 2010/015314

(56) Entgegenhaltungen:
- WO-A1-02/16298
- WO-A1-91/02712
- WO-A1-2008/125241
- JP-A- 2005 330 225
- US-A- 3 342 858

## Beschreibung

Arylpolyglykolcarbonsäuren (Ethercarbonsäuren), d. h. organische Carbonsäuren, die neben der Carboxylfunktion eine oder mehrere Etherbrücken tragen, bzw. deren Alkali- oder Aminsalze, sind als milde Detergenzien mit hohem Kalkseifendispergiervermögen bekannt. Sie finden sowohl in Waschmittel- und Kosmetikformulierungen als auch in technischen Anwendungen, wie z. B. Metallbearbeitungsflüssigkeiten und Kühlschmiermittel, Verwendung.

Ethercarbonsäuren werden gemäß dem Stand der Technik entweder durch Alkylierung von Arylpolyglykolen mit Chloressigsäurederivaten (Williamsonsche Ethersynthese) oder aus den gleichen Ausgangsprodukten durch Oxidation mit verschiedenen Reagenzien (Luftsauerstoff, Hypochlorit, Chlorit) unter Katalyse mit verschiedenen Katalysatoren dargestellt. Die Williamsonsche Ethersynthese stellt vor allem aufgrund der Kosten-Wirkung-Beziehung das technisch geläufigste Verfahren für die Herstellung von Ethercarbonsäuren dar, jedoch besitzen durch dieses Verfahren hergestellte Produkte noch gravierende Mängel in Bezug auf die Handhabbarkeit für den Anwender, wie beispielsweise Löslichkeitsverhalten, Aggregatzustand bei niedrigen Temperaturen und Lagerstabilität.

Diese Mängel sind im Wesentlichen auf verfahrensbedingte Nebenbestandteile zurückzuführen. So werden trotz Verwendung von Überschüssen des entsprechenden Chloressigsäurederivats nur Umsätze von ca. 70 - 85 % erreicht, so dass Restmengen von Oxethylat und Fettalkohol, der dem Oxethylat zugrunde liegt, im Endprodukt verbleiben. Des Weiteren entstehen durch den zu verwendenden Überschuss des Chloressigsäurederivats Folgeprodukte, wie beispielsweise Glykolsäure, Diglykolsäure und deren Derivate, die eine wesentliche Ursache für die Alterung der Produkte sind und gegebenenfalls Probleme beim Löslichkeitsverhalten hervorrufen können.

Ein weiterer Nachteil der Williamsonschen Synthese besteht in der hohen Belastung der Reaktionsprodukte durch Natriumchlorid, das in wässrigen Lösungen eine wesentliche Ursache für Lochfraß-Korrosion darstellt. Außerdem gelangt das gebildete Natriumchlorid in das Reaktionsabwasser und stellt dort für biologische Kläranlagen ein Problem dar, da Kochsalz die Reinigungsleistung solcher Anlagen beeinträchtigen kann.

Die direkte Oxidation von Alkoholoxethylaten zu Ethercarbonsäuren gelingt mithilfe von Platin-Katalysatoren, wie z. B. in US-3 342 858 beschrieben. Platin kann sowohl als Suspension verwendet werden, als auch auf einem Trägermaterial wie Kohlenstoff aufgebracht sein. Die Oxidation wird in alkalischer Lösung bei einer Temperatur von 20 bis 75 °C und einem maximalen Druck von 3 bar durchgeführt. Nachteil dieses Verfahrens sind die sehr verdünnten Lösungen (3 bis 12 %ige wässrige Lösungen), die teilweise langen Reaktionszeiten von bis zu 24 Stunden und die damit verbundene geringe Raum-Zeit-Ausbeute. Nachteilig sind bei den verwendeten Platin-Katalysatoren ebenfalls die geringen Selektivitäten; die Ausbeuten betragen nach destillativer Aufarbeitung nur ca. 68 bis 89 %.

Überraschend wurde nun gefunden, dass Ethercarbonsäuren und deren Salze auch durch direkte Oxidation von Arylpolyglykolen mit Luftsauerstoff oder Reinsauerstoff mittels goldhaltigen Katalysatoren in hoher Ausbeute zugänglich sind.

Gegenstand der vorliegenden Erfindung ist daher ein Verfahren zur Herstellung von Verbindungen der Formel (I) 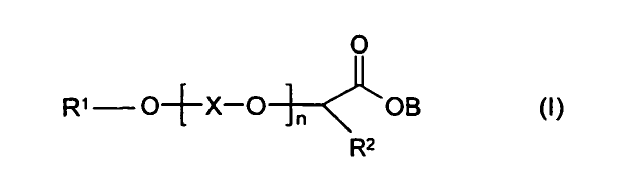
- R¹: eine aromatische Gruppe mit 6 bis 200 Kohlenstoffatomen
- R²: Wasserstoff, einen linearen oder verzweigten Alkylrest mit 1 bis 22 Kohlenstoffatomen, einen ein- oder mehrfach ungesättigten linearen oder verzweigten Alkenylrest mit 2 bis 22 Kohlenstoffatomen, oder einen Arylrest mit 6 bis 12 Kohlenstoffatomen,
- X: ein Alkylenrest mit 2 bis 4 Kohlenstoffatomen,
- n: eine Zahl zwischen 0 und 100,
- B: ein Kation oder Wasserstoff
bedeuten, und/oder der entsprechenden protonierten Carbonsäuren, indem ein oder mehrere Verbindungen der Formel (II) 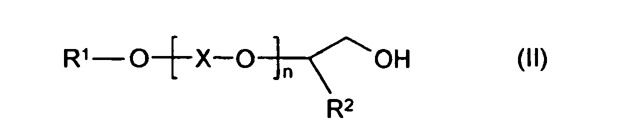 worin R¹, R², X und n die oben angegebene Bedeutung besitzen, mit Sauerstoff oder Sauerstoff enthaltenden Gasen in Gegenwart eines goldhaltigen Katalysators, welcher Gold und ein weiteres Element der Gruppe VIII im Gewichtsverhältnis An : Gruppe VIII-Metall = 70 : 30 bis 95 : 5 enthält, und mindestens einer alkalischen Verbindung oxidiert werden.

R¹ ist vorzugsweise eine aromatische Gruppe mit 6 bis 24 Kohlenstoffatomen. Besonders bevorzugt ist R¹ eine reine Kohlenwasserstoffgruppe.

Die aromatischen Systeme, die in R¹ enthalten sind, können mit Alkyl- oder Alkenylgruppen substituiert sein, die 1 - 200, vorzugsweise 2 - 20, insbesondere 4 - 16 wie beispielsweise 6-12 Kohlenstoffatome enthalten.

In besonders bevorzugter Ausführungsform handelt es sich bei R¹ um eine Phenylgruppe, die mit Alkyl- oder Alkenylgruppen substituiert ist, die 1 - 200, vorzugsweise 2 - 20, insbesondere 4 - 16 wie beispielsweise 6 -12 Kohlenstoffatome enthalten. Bevorzugt handelt es sich dabei um n-, iso- und tert.-Butyl-, n- und iso-Pentyl-, n- und iso-Hexyl-, n- und iso-Octyl-, n- und iso-Nonyl-, n- und iso-Decyl-, n- und iso-Dodecyl-, Tetradecyl-, Hexadecyl-, Octadecyl-, Tripropenyl-, Tetrapropenyl-, Poly(propenyl)- und Poly(isobutenyl)reste.

Erfindungsgemäß geeignet sind insbesondere solche aromatischen Systeme R¹, die sich von Alkylphenolen mit ein oder zwei Alkylresten in ortho- und/oder para-Position zur OH-Gruppe ableiten. Besonders bevorzugt als Ausgangsmaterialien sind Alkylphenole, die am Aromaten mindestens zwei zur Kondensation mit Aldehyden befähigte Wasserstoffatome tragen und insbesondere monoalkylierte Phenole. Besonders bevorzugt sind aromatische Systeme R¹ mit einer Alkyl- oder Alkenylgruppe, die 1 - 200, vorzugsweise 2 - 20, insbesondere 4 - 16 wie beispielsweise 6 -12 Kohlenstoffatome enthält, in der para-Stellung zur phenolischen OH-Gruppe.

In einer weiteren bevorzugten Ausführungsform werden aromatische Systeme R¹ mit unterschiedlichen Alkylresten eingesetzt, beispielsweise Butylreste einerseits und Octyl-, Nonyl- und/oder Dodecylreste im molaren Verhältnis von 1:10 bis 10:1 andererseits.

Beispielhaft steht R1 für Phenyl-, Tributylphenyl-, Tristyrylphenyl-, Nonylphenyl-, Cumyl- oder Octylphenylreste.

Bevorzugt ist R² Wasserstoff oder ein C₁- bis C₄-Alkylrest.

Bei der Polyglykolkette (X-O) der Ausgangsverbindung (II) kann es sich um eine reine oder gemischte Alkoxykette mit statistischer oder blockweiser Verteilung von (X-O)-Gruppen handeln.

Als alkalische Verbindung können Carbonate, Hydroxide oder Oxide in dem erfindungsgemäßen Verfahren verwendet werden. Bevorzugt sind die Hydroxide BOH.

Bei den Gegenionen B handelt es sich vorzugsweise um Alkalimetallkationen, ausgewählt aus Kationen der Alkalimetalle Li, Na, K, Rb und Cs. Besonders bevorzugt sind die Kationen der Alkalimetalle Na und K. Als alkalische Verbindung im erfindungsgemäßen Verfahren sind die Hydroxide von Li, Na, K, Rb und Cs besonders bevorzugt.

Der goldhaltige Katalysator ist ein gemischter Katalysator, der neben Gold weitere Metalle der Gruppe VIII enthält. Bevorzugt sind als Katalysatoren Goldkatalysatoren, die zusätzlich mit einem der Metalle aus der Gruppe VIII dotiert sind. Besonders bevorzugt ist die Dotierung mit Platin oder Palladium.

Vorzugsweise sind die Metalle auf Trägern aufgebracht. Bevorzugte Träger sind Aktivkohle und oxidische Träger, vorzugsweise Titandioxid, Cerdioxid oder Aluminiumoxid. Solche Katalysatoren können nach den bekannten Methoden wie Incipient Wetness (IW) oder Deposition-Precipitation (DP) wie z. B. in L. Prati, G. Martra, Gold Bull. 39 (1999) 96 und S. Biella, G.L. Castiglioni, C. Fumagalli, L. Prati, M. Rossi, Catalysis Today 72 (2002) 43-49 oder L. Prati, F. Porta, Applied catalysis A: General 291 (2005) 199-203 beschrieben, hergestellt werden.

Der Katalysator enthält vorzugsweise 0,1 bis 5 Gew.-% Gold und 0,1 bis 3 Gew.-% eines Gruppe VIII-Metalls, bevorzugt Platin oder Palladium. Besonders bevorzugt sind solche Katalysatoren, die 0,5 bis 3 Gew.-% Gold enthalten. Das Gewichtsverhältnis Gold/Gruppe VIII Metall, insbesondere Gold/Platin oder Gold/Palladium, beträgt 70 : 30 bis 95 : 5.

Enthält der Katalysator Nanogold und ein weiteres Metall, so handelt es sich vorzugsweise um 0,1 bis 5 Gew.-% Nanogold und 0,1 bis 2 Gew.-% eines Gruppe VIII-Metalls, bevorzugt Platin oder Palladium. Besonders bevorzugt sind solche Katalysatoren, die 0,5 bis 3 Gew.-% Nanogold enthalten. Das bevorzugte Gewichtsverhältnis Nanogold/Gruppe VIII Metall, insbesondere Nanogold/Platin oder Nanogold/Palladium, beträgt 70 : 30 bis 95 : 5.

Das erfindungsgemäße Verfahren wird vorzugsweise in Wasser durchgeführt.

Die Oxidationsreaktion wird bei einer Temperatur von 30 bis 200 °C, bevorzugt zwischen 80 und 150 °C durchgeführt.

Der pH-Wert während der Oxidation liegt bevorzugt zwischen 8 und 13, besonders bevorzugt zwischen 9 und 11.

Der Druck bei der Oxidationsreaktion ist vorzugsweise im Vergleich zu Atmosphärendruck erhöht.

Bei der Reaktion im alkalischen Medium entstehen zunächst die Alkalisalze (B = Li, Na, K, Rb, Cs) der Carbonsäuren, bevorzugt die Natrium- oder Kaliumsalze. Zur Herstellung der freien Ethercarbonsäure (d. h. B = Wasserstoff) werden die erhaltenen Ethercarboxylate der Formel (I) mit Säuren umgesetzt. Bevorzugte Säuren sind Salz- und Schwefelsäure.

Das erfindungsgemäße Verfahren ergibt vorzugsweise Lösungen von Carboxylaten der Formel (I) mit nur noch geringen Restgehalten an Arylpolyglykolen der Formel (II) von < 10 Gew.%, bevorzugt < 5 Gew.-%, besonders bevorzugt < 2 Gew.-%.

### Beispiele

### Beispiel 1:

In einen 2-Liter-Druckautoklaven mit Begasungsrührer werden 1 Liter einer wässrigen, 10 gew.%igen Tristyrylphenolpolyethylenglykol-Lösung (16 EO, M_{w}= 1100 g/mol) gegeben. Nach Zugabe von 10g eines Nanogoldkatalysators (0,9 Gew.-% Gold und 0,1 Gew.-% Platin auf Cerdioxid, Teilchengröße 4 bis 8 nm) wird die Suspension mit Natronlauge auf pH 10 eingestellt und auf 120 °C aufgeheizt. Nach Erreichen der Reaktionstemperatur wird der Reaktionslösung Sauerstoff mit einem Druck von 10 bar aufgepresst und durch Nachpressen auf diesem Druck gehalten. Während der gesamten Reaktionszeit wird mittels eines Autotitrators der pH-Wert der Mischung mit Natronlauge auf 10 gehalten. Nach 4 Stunden wird der Reaktor abgekühlt, entspannt und der Katalysator durch Filtration von der Reaktionslösung abgetrennt. Die Lösung zeigt einen Gehalt von ca. 10 Gew.-% Tristyrylphenolpolyethylenglykolcarboxylat, Tristyrylphenolpolyethylenglykol ist nicht mehr nachweisbar.

### Beispiel 2:

In einen 2-Liter-Druckautoklaven mit Begasungsrührer werden 1 Liter einer wässrigen 10 gew.-%igen Nonylphenolpolyethylenglykol-Lösung (6 EO, M_{W} = 490 g/mol) gegeben. Nach Zugabe von 10 g eines Goldkatalysators (0,9 Gew.-% Gold und 0,1 Gew.-% Platin auf Titandioxid, Teilchengröße 4 bis 8 nm) wird die Suspension mit Natronlauge auf pH 11 eingestellt und auf 110 °C aufgeheizt. Nach Erreichen der Reaktionstemperatur wird der Reaktionslösung Sauerstoff mit einem Druck von 8 bar aufgepresst und durch Nachpressen auf diesem Druck gehalten. Während der gesamten Reaktionszeit wird mittels eines Autotitrators der pH-Wert der Mischung mit Natronlauge auf 11 gehalten. Nach 2 Stunden wird der Reaktor abgekühlt, entspannt und der Katalysator durch Filtration von der Reaktionslösung abgetrennt. Die Lösung zeigt einen Gehalt von ca. 10 Gew.-% Nonylphenolpolyglykolcarboxylat, Nonylphenolethoxylat ist nicht mehr nachweisbar.

### Beispiel 3:

In einen 2-Liter-Druckautoklaven mit Begasungsrührer werden 1 Liter einer wässrigen 10 gew.-%igen Tri-sec-butylphenolpolyethylenglykol-Lösung (6 EO, M_{W} = 530 g/mol) gegeben. Nach Zugabe von 10 g eines Goldkatalysators (0,9 Gew.-% Gold und 0,1 Gew.-% Platin auf Titandioxid, Teilchengröße 4 bis 8 nm) wird die Suspension mit Natronlauge auf pH 11 eingestellt und auf 100 °C aufgeheizt. Nach Erreichen der Reaktionstemperatur wird der Reaktionslösung Sauerstoff mit einem Druck von 8 bar aufgepresst und durch Nachpressen auf diesem Druck gehalten. Während der gesamten Reaktionszeit wird mittels eines Autotitrators der pH-Wert der Mischung mit Natronlauge auf 11 gehalten. Nach 3 Stunden wird der Reaktor abgekühlt, entspannt und der Katalysator durch Filtration von der Reaktionslösung abgetrennt. Die Lösung zeigt einen Gehalt von ca. 10 Gew.-% Tri-sec-butylphenolpolyethylenglykolcarboxylat, Tri-sec-butylphenolpolyethylenglykol ist nicht mehr nachweisbar.

## Patentansprüche

1. Verfahren zur Herstellung von Verbindungen der Formel (I) 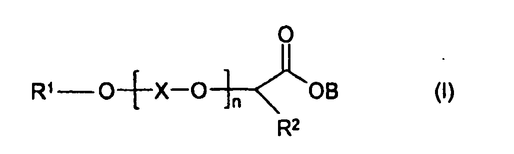
R¹ eine aromatische Gruppe mit 6 bis 200 Kohlenstoffatomen
R² Wasserstoff, einen linearen oder verzweigten Alkylrest mit 1 bis 22 Kohlenstoffatomen, einen ein- oder mehrfach ungesättigten linearen oder verzweigten Alkenylrest mit 2 bis 22 Kohlenstoffatomen, oder einen Arylrest mit 6 bis 12 Kohlenstoffatomen,
X ein Alkylenrest mit 2 bis 4 Kohlenstoffatomen,
n eine Zahl zwischen 0 und 100,
B ein Kation oder Wasserstoff
bedeuten, und/oder der entsprechenden protonierten Carbonsäuren, indem ein oder mehrere Verbindungen der Formel (II) 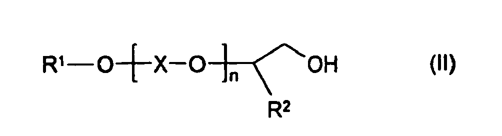 worin R¹, R², X und n die oben angegebene Bedeutung besitzen, mit Sauerstoff oder Sauerstoff enthaltenden Gasen in Gegenwart eines goldhaltigen Katalysators, welcher Gold und ein weiteres Element der Gruppe VIII im Gewichtsverhältnis Au : Gruppe VIII-Metall = 70 : 30 bis 95 : 5 enthält, und mindestens einer alkalischen Verbindung oxidiert werden.

2. Verfahren nach Anspruch 1, worin der goldhaltige Katalysator ein Nanogold-Katalysator mit einer mittleren Teilchengröße von 1 bis 50 nm ist.

3. Verfahren nach Anspruch 2, worin der Nanogold-Katalysator auf einem oxidischen Träger oder auf Kohlenstoff aufgebracht ist.

4. Verfahren nach Anspruch 3, worin der oxidische Träger aus Titandioxid, Aluminumoxid oder Cerdioxid besteht.

5. Verfahren nach einem oder mehreren der Ansprüche 2 bis 4, worin der Nano-Gold-Katalysator 0,1 bis 5 Gew.-% Nanogold enthält.

6. Verfahren nach einem oder mehreren der Ansprüche 1 bis 5, worin R¹ eine aromatische Gruppe mit 6 bis 24 Kohlenstoffatomen ist.

7. Verfahren nach einem oder mehreren der Ansprüche 1 bis 6, worin R¹ eine Kohlenwasserstoffgruppe ist.

8. Verfahren nach einem oder mehreren der Ansprüche 1 bis 7, worin die in R¹ enthaltenen aromatischen Systeme mit Alkyl- oder Alkenylgruppen mit 1 bis 200 Kohlenstoffatomen substituiert sind.

9. Verfahren nach einem oder mehreren der Ansprüche 1 bis 8, worin R¹ ausgewählt ist aus Phenyl-, Tributylphenyl-, Tristyrylphenyl-, Nonylphenyl- oder Octylphenylgruppen, sowie aus Phenylgruppen, die mit n-, iso- und tert.-Butyl-, n- und iso-Pentyl-, n- und iso-Hexyl-, n- und iso-Octyl-, n- und iso-Nonyl-, n- und iso-Decyl-, n- und iso-Dodecyl-, Tetradecyl-, Hexadecyl-, Octadecyl-, Tripropenyl-, Tetrapropenyl-, Poly(propenyl)- und Poly(isobutenyl)resten substituiert sind.

10. Verfahren nach einem oder mehreren der Ansprüche 1 bis 9, worin R² Wasserstoff oder ein C₁₋ bis C₄-Alkylrest ist.

11. Verfahren nach einem oder mehreren der Ansprüche 1 bis 10, worin B Wasserstoff oder ein Kation der Alkalimetalle Li, Na, K, Rb und Cs ist.

## Claims

1. A method for producing compounds of the formula (I) 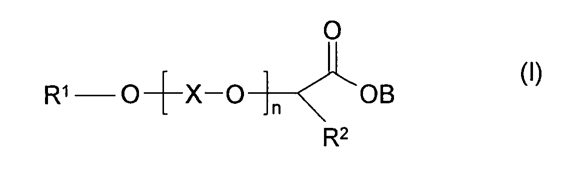
R¹ is an aromatic group having 6 to 200 carbon atoms,
R² is hydrogen, a linear or branched alkyl radical having 1 to 22 carbon atoms, a mono- or polyunsaturated linear or branched alkenyl radical having 2 to 22 carbon atoms, or an aryl radical having 6 to 12 carbon atoms,
X is an alkylene radical having 2 to 4 carbon atoms,
n is a number between 0 and 100,
B is a cation or hydrogen,
and/or of the corresponding protonated carboxylic acids by oxidizing one or more compounds of the formula (II) 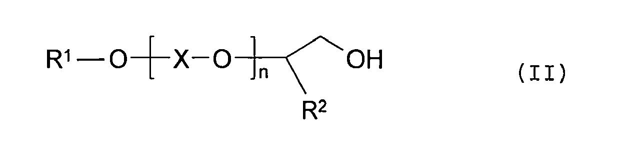 in which R¹, R², X and n have the meaning given above, with oxygen or gases containing oxygen in the presence of a gold-containing catalyst, which comprises gold and a further element of group VIII in the weight ratio Au:group VIII metal = 70:30 to 95:5, and at least one alkaline compound.

2. The method as claimed in claim 1, wherein the gold-containing catalyst is a nanogold catalyst with an average particle size of from 1 to 50 nm.

3. The method as claimed in claim 2, wherein the nanogold catalyst is applied to an oxidic support or to carbon.

4. The method as claimed in claim 3, wherein the oxidic support comprises titanium dioxide, aluminum oxide or cerium dioxide.

5. The method as claimed in one or more of claims 2 to 4, wherein the nanogold catalyst comprises 0.1 to 5% by weight of nanogold.

6. The method as claimed in one or more of claims 1 to 5, wherein R¹ is an aromatic group having 6 to 24 carbon atoms.

7. The method as claimed in one or more of claims 1 to 6, wherein R¹ is a hydrocarbon group.

8. The method as claimed in one or more of claims 1 to 7, wherein the aromatic systems present in R¹ are substituted with alkyl or alkenyl groups having 1 to 200 carbon atoms.

9. The method as claimed in one or more of claims 1 to 8, wherein R¹ is selected from phenyl, tributylphenyl, tristyrylphenyl, nonylphenyl or octylphenyl groups, and also from phenyl groups which are substituted with n-, iso- and tert-butyl radicals, n- and isopentyl radicals, n- and isohexyl radicals, n- and isooctyl radicals, n- and isononyl radicals, n- and isodecyl radicals, n- and isododecyl radicals, tetradecyl radicals, hexadecyl radicals, octadecyl radicals, tripropenyl radicals, tetrapropenyl radicals, poly(propenyl) radicals and poly(isobutenyl) radicals.

10. The method as claimed in one or more of claims 1 to 9, wherein R² is hydrogen or a C₁ to C₄-alkyl radical.

11. The method as claimed in one or more of claims 1 to 10, wherein B is hydrogen or a cation of the alkali metals Li, Na, K, Rb and Cs.

## Revendications

1. Procédé pour la préparation de composés de formule (I) 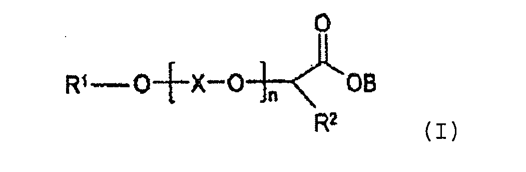 R¹ représente un groupe aromatique ayant de 6 à 200 atomes de carbone
R² représente un atome d'hydrogène, un radical alkyle linéaire ou ramifié ayant de 1 à 22 atomes de carbone, un radical alcényle linéaire ou ramifié, une ou plusieurs fois insaturé, ayant de 2 à 22 atomes de carbone, ou un radical aryle ayant de 6 à 12 atomes de carbone,
X représente un radical alkylène ayant de 2 à 4 atomes de carbone,
n représente un nombre compris entre 0 et 100,
B représente un cation ou un atome d'hydrogène, et/ou des acides carboxyliques protonés correspondants, par oxydation d'un ou plusieurs composés de formule (II) 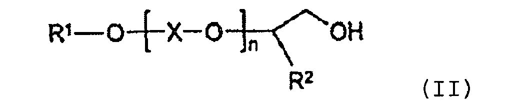 dans laquelle R¹, R², X et n ont la signification indiquée, avec de l'oxygène ou des gaz contenant de l'oxygène, en présence d'un catalyseur contenant de l'or, qui contient de l'or et un autre élément du groupe VIII en un rapport pondéral Au : métal du groupe VIII = 70 : 30 à 95 : 5, et d'au moins un composé alcalin.

2. Procédé selon la revendication 1, dans lequel le catalyseur contenant de l'or est un catalyseur au nano-or ayant une taille moyenne de particule de 1 à 50 nm.

3. Procédé selon la revendication 2, dans lequel le catalyseur au nano-or est appliqué sur un support de type oxyde ou sur du carbone.

4. Procédé selon la revendication 3, dans lequel le support de type oxyde consiste en dioxyde de carbone, oxyde d'aluminium ou oxyde de cérium.

5. Procédé selon une ou plusieurs des revendications 2 à 4, dans lequel le catalyseur au nano-or contient 0,1 à 5 % en poids de nano-or.

6. Procédé selon une ou plusieurs des revendications 1 à 5, dans lequel R¹ est un groupe aromatique ayant de 6 à 24 atomes de carbone.

7. Procédé selon une ou plusieurs des revendications 1 à 6, dans lequel R¹ est un groupe hydrocarboné.

8. Procédé selon une ou plusieurs des revendications 1 à 7, dans lequel les systèmes aromatiques contenus dans R¹ sont substitués par des groupes alkyle ou alcényle ayant de 1 à 200 atomes de carbone.

9. Procédé selon une ou plusieurs des revendications 1 à 8, dans lequel R¹ est choisi parmi les groupes phényle, tributylphényle, tristyrylphényle, nonylphényle ou octylphényle, ainsi que les groupes phényle qui sont substitués par des radicaux n-, iso- et tert-butyle, n- et isopentyle, n- et isohexyle, net iso-octyle, n- et isononyle, n- et isodécyle, n- et isododécyle, tétradécyle, hexadécyle, octadécyle, tripropényle, tétrapropényle, poly(propényle) et poly(isobutényle).

10. Procédé selon une ou plusieurs des revendications 1 à 9, dans lequel R² est un atome d'hydrogène ou un radical alkyle en C₁-C₄.

11. Procédé selon une ou plusieurs des revendications 1 à 10, dans lequel B représente un atome d'hydrogène ou un cation des métaux alcalins Li, Na, K, Rb et Cs.
